# EUROPÄISCHE PATENTANMELDUNG

(11) **EP 2 110 155 A1**
(43) Veröffentlichungstag der Anmeldung: **21.10.2009**
(21) Anmeldenummer: 09155554.0
(22) Anmeldetag: 19.03.2009
(51) Int. Cl.: A61N 1/05

(54) **Steuerbare Elektrode zur tiefen Hirnstimulation**

(30) Priorität: 19.04.2008 DE 102008019827
(71) Anmelder: Biotronik CRM Patent AG, 6341 Baar (CH)
(72) Erfinder: Flach, Erhard, 12305, Berlin (DE); Dörr, Thomas, 12437, Berlin (DE)
(74) Vertreter: Lindner-Vogt, Karin L.

(57) **Zusammenfassung**

Steuerbarer Katheter (10) mit einem länglichen Schaft (13), welcher in mehrere Schaftsegmente unterteilt ist, von denen wenigstens zwei als Kippsegmente (12) ausgebildet sind und mit jeweils einer Stellvorrichtungen so verbunden sind, dass sie um einen gewünschten Kippwinkel gegenüber der Längsachse des Schaftes (13) verkippbar sind, dadurch gekennzeichnet, dass jede der Stellvorrichtungen einzeln ansteuerbar ist und ein individuelles Kippen eines entsprechenden Kippsegmentes um einen gewünschten Kippwinkel ermöglicht.

## Beschreibung

Die Erfindung betrifft Katheter, insbesondere Zuführ- oder Stimulationskatheter, welche zur Kanalbildung im Körpergewebe eines Patienten zum Einsatz kommen. Insbesondere betrifft die vorliegende Erfindung solche Katheter, welche während der Kanalbildung im Körper des Patienten gesteuert werden können.

Aus dem Stand der Technik sind eine Vielzahl von Lösungen zur Steuerung von Kathetern bekannt. Bekannt sind insbesondere auch solche Katheter, welche über einen Zugdraht verfügen, der in Längsrichtung des Katheters so geführt und an dessen distalem Ende so befestigt ist, dass durch ein Spannen des Zugdrahtes ein bestimmter Längsabschnitt des Katheters gekrümmt wird.

Derartige Katheter werden vor allem beim Einsatz im venösen oder arterielle Gefäßsystem eines Patienten oder anderen Körperhohlräumen verwendet, wobei der Weg des Katheters beim Einführen im Großen und Ganzen durch die Geometrie der Gefäße bereits vorbestimmt ist. Die Steuerbarkeit eines derartigen Katheters dient im Wesentlichen dem Aufsuchen bestimmter Gefäßabzweigungen oder dem Aufsuchen gewünschter Areale in Körperhohlräumen.

Katheter, welche zur Kanalbildung im Körpergewebe eines Patienten ausgebildet sind, kommen zum Beispiel bei der Implantation von Elektroden zur tiefen Hirnstimulation (deep brain stimulation: DBS) im Gehirn von Patienten zum Einsatz. Beim Einführen muss der Katheter im Gehirn des Patienten den Implantationskanal erst formen, weshalb der Weg des Katheters anders als beim Einführen in das Gefäßsystem eines Patienten oder in Körperhohlräume nicht vorherbestimmt ist.

Darüber hinaus muss der Katheter einem exakt vom Chirurgen vorgegebenen Weg folgen, um zum Beispiel kritische Gewebeareale und Regionen auszusparen beziehungsweise zu umgehen.

Für diese Aufgabe ist die Steuerbarkeit herkömmlicher Katheter nicht ausreichend, insbesondere sind die in diesem Zusammenhang einstellbaren Krümmungsradien herkömmlicher Katheter nur unzureichend variierbar und ermöglichen daher keine präzise und exakte Kanalbildung. Steuerbare Katheter gemäß dem Stand der Technik üben gerade bei einem Richtungswechsel und daran anschließender Vorschub-Krafteinleitung eine nicht tolerierbare zusätzliche Normalkraft auf das Körper- in diesem Fall das Hirngewebe aus.

Die Aufgabe der vorliegenden Erfindung besteht demnach darin, ein kanalbildendes Instrument bereit zu stellen, welches eine präzise und schonende Kanalbildung im Körpergewebe eines Patienten ermöglicht und insbesondere während des Vorschiebens nach einem Richtungswechsel nur tolerierbare Normalkräfte auf das empfindliche Körpergewebe ausübt.

Erfindungsgemäß wird diese Aufgabe durch einen steuerbaren Katheter mit einem länglichen Schaft gelöst, wobei der Schaft in mehrere Schaftsegmente unterteilt ist, von denen wenigstens zwei als Kippsegmente ausgebildet sind und mit jeweils einer Stellvorrichtung so verbunden sind, dass sie um einen gewünschten Kippwinkel gegenüber der Achse des Schaftes verkippbar sind. Jede der Stellvorrichtungen ist dabei einzeln ansteuerbar und ermöglicht ein individuelles Kippen eines entsprechenden Kippsegmentes um einen gewünschten Kippwinkel.

Ein derartiges kanalbildendes Instrument ermöglicht es, zunächst das in distaler Richtung vorderste Kippsegment mit Hilfe dessen Stellvorrichtung zu verkippen und dadurch einen Richtungswechsel des Katheters einzuläuten. Beim anschließenden weiteren Vorschieben des Instrumentes um die Länge des vordersten Kippsegment ist das proximal an das vorderste Kippsegment angrenzende Kippsegment dazu ausgebildet, dem vom vordersten Kippsegment gebildeten Kanal zu folgen und dabei den Kippwinkel des vordersten Kippsegmentes zu übernehmen.

Zu diesem Zweck ist auch das proximal an das vorderste (im Folgenden auch erste) angrenzende Kippsegment mit einer separat ansteuerbaren Stellvorrichtung ausgestattet. Die Anpassung des Kippwinkels dieses zweiten Kippsegmentes an den Kippwinkel des ersten Kippsegmentes im Verlauf des Vorschiebens bewirkt, dass der erfindungsgemäße Katheter beim Vorschub dem Verlauf des schon gebildeten Kanals folgt und so keine unnötigen Normalkräfte auf das Körpergewebe ausübt.

Vorzugsweise verfügt der Katheter über eine Vielzahl von Kippsegmenten, welche jeweils einzeln ansteuerbar und verkippbar sind. Dies ermöglicht, dass nach einem Richtungswechsel nicht nur das proximal an das erste angrenzende Kippsegment, sondern bei einem weiteren Vorschub auch die jeweils folgenden Kippsegmente nacheinander so verstellbar sind, dass sie während des Vorschiebens dem Verlauf des Kanals folgen und so verhindern, dass eine nicht tolerierbare Normalkraft auf das Körpergewebe wirkt.

Vorzugsweise sind die Stellvorrichtungen der einzelnen Kippsegmente mit einer Steuervorrichtung verbunden, die eine jeweilige Einstellung der Kippwinkel kontrolliert und bei Bedarf die jeweiligen Stellvorrichtungen veranlasst, die entsprechenden Kippwinkel anzupassen.

Besonders bevorzugt verfügt der Katheter auch über einen Bewegungssensor, der eine Verschiebung des Katheters in distaler oder proximaler Richtung registriert. Auf diese Art und Weise ist es möglich, nach einem Richtungswechsel und weiterem Vorschub des Katheters eine automatische Anpassung der Kippwinkel der proximal zum ersten Kippsegment angeordnete Kippsegmente vorzunehmen, sodass die einzelnen Kippsegmente dem schon gebildeten Gewebekanal folgen.

Vorzugsweise ist der Bewegungssensor mit der Steuereinheit verbunden und dazu ausgebildet, auch ein Verschieben des Katheters in proximaler Richtung zu registrieren. Analog zu der soeben geschilderten Anpassung der Kippsegmente beim Vorschub ist der Katheter vorzugsweise beim Zurückziehen dazu ausgebildet, die einzelnen Kippsegmente automatisch anzupassen.

Gemäß einer bevorzugten Ausführungsvariante ist die Steuereinheit dazu ausgebildet, während des Vorschubs oder während des Zurückziehens des Katheters den Kippwinkel der Kippsegmente langsam zu verändern.

Gemäß einer weiteren Ausführungsform der vorliegenden Erfindung verfügen die Stellvorrichtungen der einzelnen Kippsegmente über Zugdrähte bzw. Zugseile, mit denen die Kippwinkel der einzelnen Kippsegmente eingestellt werden können.

Vorzugsweise umfassen die Stellvorrichtungen jedoch piezoelektrische Aktuatoren, mit denen die Kippwinkel der Kippsegmente verändert werden können.

Besonders bevorzugt verfügen die Stellvorrichtungen über wenigstens zwei ringförmig angeordnete piezoelektrische Aktuatoren, von denen bevorzugt jeder einzeln ansteuerbar ist.

In einer Ausführungsform als Stimulationskatheter verfügt das erfindungsgemäße Instrument über wenigstens ein Kippsegment, welches eine Elektrode aufweist.

Gemäß einer bevorzugten Ausführungsvariante ist ausschließlich das in distaler Richtung vorderste Kippsegment direkt vom Operateur steuerbar und die Verkippung der übrigen Kippsegmente wird automatisch durch die Steuervorrichtung geregelt.

Der Katheterschaft selbst ist gemäß verschiedenen Ausführungsvarianten aus einem Rohr oder aus einem elastischen Vollmaterial gebildet und hat gemäß einer besonders bevorzugten Ausführungsvariante einen Durchmesser von weniger als 3,3 mm.

Gemäß einer bevorzugten Ausführungsvariante sind die einzelnen Kippsegmente durch quer zur Katheterlängsachse ausgerichtete Einkerbungen oder Einschnitte in dem Schaftmaterial gebildet. In den Einkerbungen bzw. Einschnitten sind vorzugsweise die Stellvorrichtungen untergebracht, die eine Verkippung der Kippsegmente bewirken. Die Einkerbungen bzw. Einschnitte haben vorzugsweise eine Tiefe und eine Breite, die einen ausreichend kleinen Krümmungsradius des Katheters bei maximalem Kippwinkel ermöglichen und gleichzeitig noch eine ausreichende Stabilität des Katheters gewährleisten.

Gemäß verschiedenen Ausgestaltungen können die Einschnitte bzw. Einkerbungen ausschließlich einseitig des Katheters, zwei- oder mehrseitig oder auch durch radiale Einkerbungen ausgebildet sein. Je nach Ausführungsform ergibt sich dadurch ein unterschiedlicher Freiheitsgrad in der Steuerbarkeit des Katheters.

Weitere Aspekte des erfindungsgemäßen Katheters und verschiedene Ausführungsvarianten sind anhand der Zeichnungen im Folgenden näher erläutert.

### Es zeigen:

Figuren 1a), 1b) und 1c) drei zeitlich versetzte schematische Darstellungen des Katheters zur Kanalbildung im Körpergewebe eines Patienten;

Figur 2 eine erste Ausführungsform des erfindungsgemäßen Katheters mit durch Kerben im Katheterschaft definierten Kippsegmenten und Zugdrähte umfassenden Stellvorrichtungen.

Figur 3 eine zweite Ausführungsform des erfindungsgemäßen Katheters mit durch Kerben im Katheterschaft definierten Kippsegmenten und piezostriktive Elemente umfassenden Stellvorrichtungen.

Figur 4 eine Detailansicht einer dritten Ausführungsform des erfindungsgemäßen Katheters, welcher über Kippsegmente verfügt, die über Aktuatoren miteinander verbunden sind.

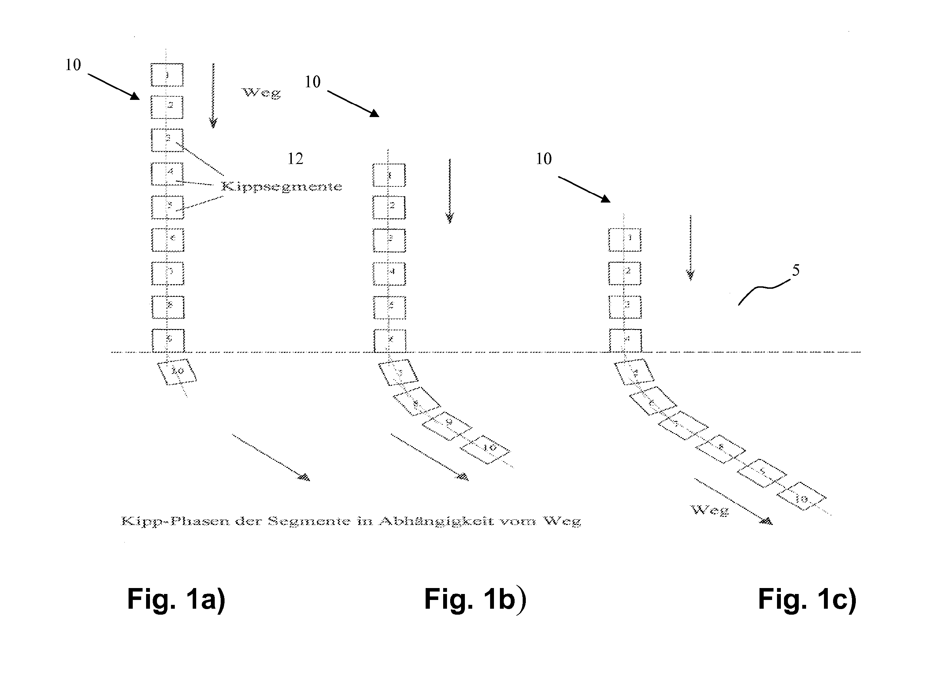

Die Figuren 1a), 1b) und 1c) zeigen den erfindungsgemäßen Katheter 10 bei der Bildung eines gekrümmten Kanals im Körpergewebe 5 eines Patienten. Dabei zeigen die drei Figuren den erfindungsgemäßen Katheter 10 in drei zeitlich aufeinander folgenden Positionen bei der Kanalbildung.

Der hier schematisch dargestellte Katheter 10 verfügt über eine Vielzahl von Kippsegmenten 12.

Direkt unter der Oberfläche des Körpergewebes 5 ist gemäß der Darstellung 1a) vorgesehen, einen gekrümmten Kanal zu bilden. Um beim Vorschub des Katheters einen gekrümmten Kanal zu erzeugen, ist das am distalen Ende angeordnete erste Kippsegment 12 des Katheters 10 bereits in einem Kippwinkel zur Längsachse des Katheters 10 ausgelenkt.

Figur 1b) zeigt den weiteren Vorschub des Katheters 10 im Körpergewebe 5 des Patienten. Wie in dieser Figur zu erkennen ist, sind die einzelnen im Körpergewebe 5 des Patienten befindlichen Kippsegmente 12 des Katheters 10 jeweils in einem unterschiedlichen Kippwinkel zur Längsachse des Katheters 10 gekippt. Weil alle Kippsegmente 12 einzeln ansteuerbar sind, wird dem Operateur ermöglicht, einen beliebig gekrümmten Kanal im Körpergewebe 5 eines Patienten zu bilden. Dabei sind die Kippsegmente 12 über hier nicht dargestellte Stellvorrichtungen steuerbar.

In der Figur 1b) ist zu erkennen, dass der Katheter 10 schon soweit im Körpergewebe 5 des Patienten vorgeschoben ist, dass sich bereits das vierte Kippsegment (vom distalen Ende her gesehen) in der Position befindet, in der sich in Figur 1a) das erste Kippsegment befunden hat.

Erkennbar besitzt das vom distalen Ende her gesehen vierte Kippsegment den gleichen Kippwinkel, den das erste Kippsegment 12 des Katheters 10 in der Figur 1a) besessen hat. Während gemäß der Figur 1a) das erste Kippsegment 12 des Katheters 10 und das proximal daran angrenzende zweite Kippsegment 12 gegenüber der Längsachse des Katheters 10 unterschiedliche Kippwinkel besaßen, ist in Figur 1b) zu erkennen, dass das erste Kippsegment 12 und das proximal daran angrenzende zweite Kippsegment 12 des Katheters 10 gegenüber der Längsachse des Katheters nun den gleichen Kippwinkel aufweisen.

Wie aus Figur 1c) zu erkennen ist, bewirkt ein weiterer Vorschub des Katheters 10, der gemäß Figur 1b) über ein erstes und ein zweites Kippsegment 12 mit gleichem Kippwinkel verfügt, dass ein gerader Abschnitt des Kanals im Körpergewebe 5 des Patienten gebildet wird.

Das erste Kippsegment 12 des Katheters 10 kann direkt vom Operateur angesteuert und gekippt werden. Die übrigen Kippsegmente 12 werden durch eine hier nicht dargestellt Steuereinheit automatisch ausgerichtet.

Durch einen hier nicht erkennbaren Sensor wird der Vorschub des Katheters 12 im Körpergewebe 5 eines Patienten registriert und an die mit dem Katheter 10 verbindbare Steuereinheit weitergegeben.

Die Steuereinheit bewirkt, dass alle proximal zum ersten Kippsegment 12 angeordneten Kippsegmente 12 so ausgerichtet werden, dass sie beim Vorschub der Krümmung des schon gebildeten Kanals folgen.

Während des Vorschubs des Katheters 10 um die Länge eines Kippsegmentes 12 nimmt ein jeweiliges Kippsegment einen Kippwinkel an, den das jeweils distal angrenzende Kippsegment 12 an der gleichen Stelle im Gewebekanal zuvor aufgewiesen hat.

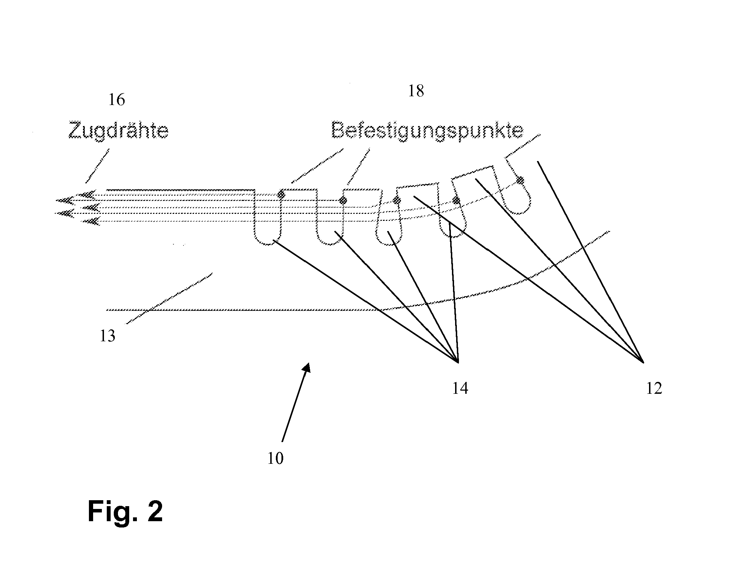

In Figur 2 ist der erfindungsgemäße Katheter 10 gemäß einer ersten Ausführungsform dargestellt, wonach er über Kippsegmente verfügt, die in dem Katheterschaft 13 durch quer zur Längsachse des Katheters 10 angeordnete Einkerbungen 14 definiert sind. Jedes der Kippsegmente 12 verfügt dabei über Befestigungspunkte 18, welche mit jeweils einem Zugdraht 16 verbunden sind.

Durch ein entsprechendes Verkürzen eines beliebigen Zugdrahtes 16 wird auf den entsprechenden Befestigungspunkt 18 das mit dem verkürzten Zugdraht 16 verbundene Kippsegment 12 gegenüber der Längsachse des Katheters 10 ausgelenkt. Der maximal mögliche Kippwinkel, welcher den minimal möglichen Radius eines zu bildenden Kanals im Gewebe 5 eines Patienten bedeutet, ist durch die Breite und Tiefe der Einkerbungen 14 gegeben. Je breiter und tiefer die Einkerbungen 14 im Katheterschaft 13 des Katheters 10 sind, desto weiter können die Zugdrähte 16 verkürzt werden und desto stärker können die einzelnen Kippsegmente 12 durch das Verkürzen der Zugdrähte 16 verkippt werden. Durch das Verkippen der Kippsegmente 12 wird ein Krümmungsradius des Katheterschafts 13 gebildet. In der hier dargestellten Ausführungsform ist der Katheterschaft 13 lediglich von einer Seite her eingekerbt und lateral ausschließlich einseitig zur Katheterachse über Befestigungspunkte 18 mit Zugdrähten 16 verbunden, sodass bei dem hier dargestellten erfindungsgemäßen Katheter 10 ausschließlich eine Krümmung in eine Richtung möglich ist.

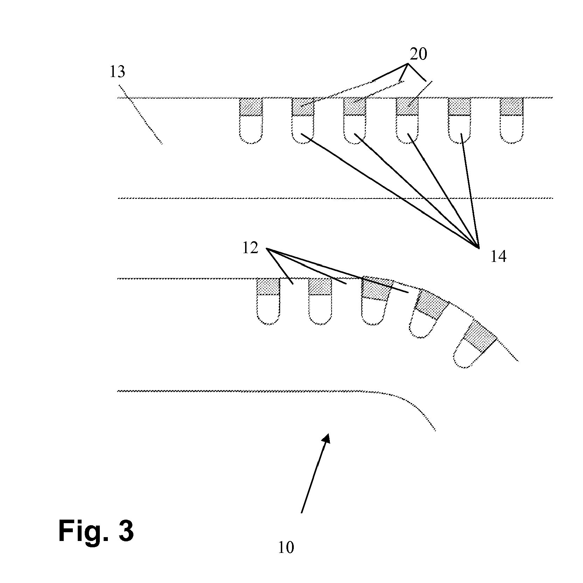

Die in Figur 3 dargestellte Ausführungsform des erfindungsgemäßen Katheters 10 entspricht der Ausführungsform des Katheters 10 gemäß Figur 5 mit dem Unterschied, dass hier die Stellvorrichtung zum Einstellen der Kippwinkel der einzelnen Kippsegmente 12 des Katheters 10 piezostriktive Aktuatoren 20 umfassen.

Auch in dieser Ausführungsform sind alle Aktuatoren 20 jeweils einzeln ansteuerbar und ermöglichen eine Anpassung des entsprechenden Kippwinkels jedes einzelnen Kippsegmentes 12. Anders als eine Stellvorrichtung mittels Zugdrähten sind die piezostriktiven Aktuaktoren 20 dazu ausgebildet, die Einkerbungen 14 des Katheterschafts 13 bei Bedarf aufweiten zu können, sodass eine Biegung des Katheterschafts 13 zu der den Einkerbungen gegenüberliegenden Seite des Katheterschafts 13 erfolgt.

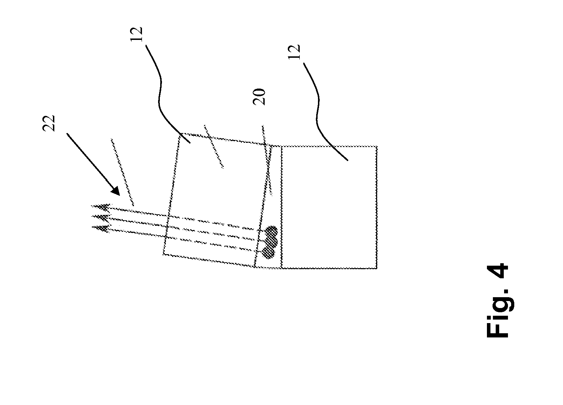

Figur 4 zeigt eine Detailansicht einer weiteren Ausführungsform des erfindungsgemäßen Katheters 10, welcher über zylindrische Kippsegmente 12 verfügt, welche über zylindrische piezostriktive Aktuatoren miteinander verbunden sind.

Der zwischen den beiden Kippsegmenten 12 befindliche Aktuator 20 ist über mehrere Steuerleitungen 22 ansteuerbar, wodurch eine Verkippung der Kippsegmente 12 erfolgen kann.

## Patentansprüche

1. Steuerbarer Katheter (10) mit einem länglichen Schaft (13), welcher in mehrere Schaftsegmente unterteilt ist, von denen wenigstens zwei als Kippsegmente (12) ausgebildet sind und mit jeweils einer Stellvorrichtungen so verbunden sind, dass sie um einen gewünschten Kippwinkel gegenüber der Längsachse des Schaftes (13) verkippbar sind, **dadurch gekennzeichnet, dass** jede der Stellvorrichtungen einzeln ansteuerbar ist und ein individuelles Kippen eines entsprechenden Kippsegmentes (12) um einen gewünschten Kippwinkel ermöglicht.

2. Steuerbarer Katheter (10) nach Anspruch 1, **dadurch gekennzeichnet, dass** die Stellvorrichtungen mit einer Steuervorrichtung verbunden sind, die eine jeweilige Einstellung der Kippwinkel der einzelnen Kippsegmente (12) kontrolliert und anpasst.

3. Steuerbarer Katheter (10) nach einem der Ansprüche 1 oder 2, **dadurch gekennzeichnet, dass** der Katheter (10) über einen Bewegungssensor verfügt, der eine Verschiebung des Katheters (10) in distaler und/oder in proximaler Richtung registriert.

4. Steuerbarer Katheter (10) nach Anspruch 3, **dadurch gekennzeichnet, dass** bei einer Verschiebung des Katheters (10) in distaler Richtung um die Länge eines Kippsegmentes (12), ein jeweiliges Kippsegment (12) einen Kippwinkel annimmt, den das jeweils distal angrenzende Kippsegment (12) an der gleichen Stelle im Gewebekanal zuvor aufgewiesen hat.

5. Steuerbarer Katheter (10) nach Anspruch 3, **dadurch gekennzeichnet, dass** bei einer Verschiebung des Katheters (10) in proximaler Richtung um die Länge eines Kippsegmentes (12) ein jeweiliges Kippsegment einen Kippwinkel annimmt, den das jeweils proximal angrenzende Kippsegment 12 an der gleichen Stelle im Gewebekanal zuvor aufgewiesen hat.

6. Steuerbarer Katheter (10) nach einem der Ansprüche 4 oder 5, **dadurch gekennzeichnet, dass** eine Veränderung der Kippwinkel der Kippsegmente (12) während des Verschiebens kontinuierlich stattfindet.

7. Steuerbarer Katheter (10) nach einem der vorangehenden Ansprüche, welcher einen Durchmesser von weniger als 3,3 mm aufweist.

8. Steuerbarer Katheter (10) nach einem der vorangehenden Ansprüche, **dadurch gekennzeichnet, dass** die Kippwinkel der Kippsegmente (12) mit Hilfe von Zugdrähten (16) einstellbar sind.

9. Steuerbarer Katheter (10) nach einem der vorangehenden Ansprüche, **dadurch gekennzeichnet, dass** die Stellvorrichtungen piezoelektrische Aktuatoren (20) umfassen.

10. Steuerbarer Katheter (10) nach Anspruch 9, **dadurch gekennzeichnet, dass** jede Stellvorrichtung über wenigstens zwei ringförmig angeordnete Piezoaktuatoren (20) verfügt.

11. Steuerbarer Katheter (10) nach Anspruch 10, **dadurch gekennzeichnet, dass** jeder der Piezoaktuatoren (20) einzeln ansteuerbar ist.

12. Steuerbarer Katheter (10) nach einem der vorangehenden Ansprüche, **dadurch gekennzeichnet, dass** wenigstens eines der Kippsegmente (12) über wenigstens eine Elektrode verfügt.

13. Steuerbarer Katheter (10) nach einem der Ansprüche 2 bis 12, **dadurch gekennzeichnet, dass** ausschließlich das in distaler Richtung vorderste Kippsegment (12) direkt vom Operateur steuerbar ist und die Verkippung der übrigen Kippsegmente (12) automatisch durch die Steuervorrichtung geregelt wird.

14. Steuerbarer Katheter (10) nach einem der vorangehenden Ansprüche, **dadurch gekennzeichnet, dass** der Katheter (10) ein Zuführkatheter ist.

15. Steuerbarer Katheter (10) nach einem der vorangehenden Ansprüche, **dadurch gekennzeichnet, dass** der Katheter (10) ein Stimulationskatheter ist.
